Europäisches Patentamt

European Patent Office

Office européen des brevets

(19)

(11) Numéro de publication : **0 299 877 B1**

(12) **FASCICULE DE BREVET EUROPEEN**

(45) Date de publication du fascicule du brevet :
**11.03.92 Bulletin 92/11**

(51) Int. Cl.⁵ : **A61K 31/135, A61K 9/22**

(21) Numéro de dépôt : **88401846.6**

(22) Date de dépôt : **15.07.88**

(54) **Comprimés de type à matrice hydrophile à base de salbutamol et leur procédé de préparation.**

Le dossier contient des informations techniques présentées postérieurement au dépôt de la demande et ne figurant pas dans le présent fascicule.

(30) Priorité : **16.07.87 FR 8710048**

(43) Date de publication de la demande :
**18.01.89 Bulletin 89/03**

(45) Mention de la délivrance du brevet :
**11.03.92 Bulletin 92/11**

(84) Etats contractants désignés :
**AT BE CH DE ES FR GB GR IT LI LU NL SE**

(56) Documents cités :
**EP-A- 0 109 320**
**EP-A- 0 205 282**
**EP-A- 0 248 548**

(56) Documents cités :
**WO-A-85/04100**
**CHEMICAL ABSTRACTS, vol. 107, no. 14, 5 octobre 1987, page 346, résumé no. 121004r, Columbus, Ohio, US; H.L. BHALLA et al.: "An improvised controlled-release tablet of salbutamol sulfate"**

(73) Titulaire : **PIERRE FABRE MEDICAMENT**
**45, Place Abel Gance**
**F-92100 Boulogne (FR)**

(72) Inventeur : **Sournac, Michel**
**32, rue des Viviers Saix**
**F-81100 Castres (FR)**
Inventeur : **Bougaret, Joel**
**36, Bd Maréchal Joffre**
**F-81100 Castres (FR)**

(74) Mandataire : **Ahner, Francis et al**
**CABINET REGIMBEAU, 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne une composition pharmaceutique comportant à titre de principe actif du salbutamol ou l'un de ses dérivés et dont la formulation galénique permet une libération prolongée dans le temps de ce principe actif.

Le salbutamol ou [(ter-butylamino)-2(hydroxy-4 hydroxyméthyl-3-phényl)-1-éthanol] et ses dérivés sont connus pour agir sur les récepteurs ß₂ des muscles lisses bronchique et utérin (BSM 8010 M). Il existe en particulier de nombreuses spécialités à base de salbutamol utilisées pour soigner les patients asthmatiques.

Cependant, ce principe actif se détériore facilement et de nombreux progrès ont déjà été effectués pour la réalisation de compositions stables de salbutamol (FR 83 08722)

Des formes à libération prolongée d'un tel principe actif n'ont pas encore été, à ce jour, réalisées ou diffusées à grande échelle. De telles formes sont cependant souhaitables notamment dans le cas du traitement de l'asthme à dyspnée continue, et de ses crises.

La demande EP-248 548, qui n'est opposable qu'au titre de la nouveauté, décrit des compositions pharmaceutiques à libération contrôlée, dans lesquelles le principe actif est incorporé dans une matrice renfermant un polydextrose hydrosoluble et un excipient secondaire gras.

La demande EP-205 282 décrit des compositions pharmaceutiques muco-adhésives à libération prolongée, préparées à partir de granules comprenant un mélange de dérivés cellulosiques et d'alcools gras aliphatiques, enrobés dans un dérivé cellulosique muco-adhésif.

C'est pourquoi, la présente invention concerne des comprimés du type à matrice hydrophile, comportant une dispersion homogène de salbutamol ou de l'un de ses dérivés destiné à assurer :

– in vitro, une libération prolongée, régulière et indépendante du pH, du principe actif pendant un intervalle de temps d'environ 12 heures,

– in vivo, une cinétique d'entrée du principe actif dans l'organisme d'ordre zéro pendant un intervalle de temps d'environ 6 heures.

Une telle forme à libération prolongée convenablement dosée en principe actif (8 mg par exemple) est efficace avec une seule prise par jour. Elle permet d'améliorer la compliance du malade par une diminution du nombre de prises, tout en assurant l'équivalence thérapeutique par rapport à la posologie classique (4 comprimés de 2 mg / jour).

En outre, une prise unique vespérale est particulièrement adaptée à combattre les manisfestations nocturnes de l'asthme.

Cette libération prolongée est obtenue en incorporant dans la composition hydrophile à titre de composant gonflant, au moins un hydrocolloïde cellulosique de haut poids moléculaire, choisi parmi une ou plusieurs substances polymères hydrophiles de viscosité apparente comprise entre 0,1 et 30 Pa.s, et

un composant diluant (D) comprenant un diluant intrinsèque (DI) et un diluant épaississant (DE) hydrophile partiellement soluble,

le rapport pondéral gonflant / diluant étant compris entre 0,2 et 0,6, de préférence égal à 0,4.

De façon surprenante, une telle association DE/DI permet une prolongation de la libération du principe actif in vitro, notamment à partir de la quatrième heure et ce, malgré la grande hydrosolubilité du salbutamol.

L'association la plus performante du point de vue de cette prolongation s'est révélée être celle dans laquelle le rapport pondéral $\dfrac{DE}{D}$ est compris entre 0,1 et 0,6.

On utilise de préférence, à titre de principe actif, un sel de salbutamol, et notamment le sulfate de salbutamol.

Il est particulièrement avantageux d'avoir recours à des comprimés comportant 1 à 10 % en poids de principe actif (P.A.) par rapport au poids total des comprimés. Cette teneur est de préférence égale à 3 % en poids de principe actif par rapport au poids total du comprimé.

La présente invention se caractérise en outre par le choix des constituants suivants :

Le diluant intrinsèque (DI) est choisi parmi une ou plusieurs substances comprenant le lactose, le sorbitol, le mannitol, les phosphate ou sulfate de calcium, la silice colloïdale et/ou la cellulose microcristalline.

Le diluant épaississant (DE) est choisi parmi une ou plusieurs substances comprenant les amidons, dérivés des amidons, en particulier l'amidon de maïs prégélatinisé.

Le composant gonflant (G) est choisi parmi une ou plusieurs substances polymères hydrophiles de viscosité apparente, à 2 % poids/poids à 20°C, comprise entre 0,1 et 30 Pa.s.

La viscosité est mesurée à l'aide de tubes d'UBBELHODE.

Ces substances polymères hydrophiles sont, de préférence, choisies dans la famille des hydrocolloïdes protéiques ou cellulosiques et, notamment parmi les dérivés alginiques, les gommes naturelles, la méthylcellulose, la carboxyméthylcellulose, l'hydroxypropylcellulose et/ou la méthylhydroxypropylcellulose (MHPC).

Le polymère hydrophile tout particulièrement préféré est la méthylhydroxypropylcellulose dont la viscosité est d'environ 15 Pa.s.

Parmi les gommes naturelles, on utilise de préférence la carraghénine.

Ces polymères peuvent être utilisés seuls ou en association entre eux.

Les comprimés selon la présente invention comportent, en outre, un agent lubrifiant, tel que l'acide stéarique ou l'un de ses dérivés, et une substance colorante pharmaceutiquement acceptable : on choisit, en particulier, une substance de couleur bleue, de la famille des laques organiques de synthèse, à savoir l'indigotine (code EEC N°132).

Ainsi, un exemple de comprimé tout particulièrement préféré, selon la présente invention, est défini par la composition suivante :

```
- Sulfate de salbutamol ............................ 9,6 mg
- Méthylhydroxypropylcellulose, 15 Pa.s............. 86 mg
- Lactose ........................................ 150 mg
- Amidon de maïs prégélatinisé ..................... 70,9 mg
- Silice colloïdale ............................... 2,7 mg
- Stéarate de magnésium ........................... 2,7 mg
- Indigotine EEC N° 132 ........................... 0,1 mg
pour un comprimé terminé de ......................322,0 mg
```

Les comprimés selon la présente invention ont, de préférence, une forme oblongue, éventuellement sécable.

La présente invention s'étend au procédé permettant la fabrication et la réalisation industrielle des comprimés du type comportant une dispersion homogène de salbutamol ou de l'un de ses dérivés dans une matrice hydrophile contenant au moins un composant gonflant et un composant diluant.

Ce procédé se caractérise par les étapes successives suivantes :

1) tamisage des différents composants, gonflant et diluant, et du principe actif ;

2) mélange de ces composants, gonflant et diluant, et du principe actif à raison d'un rapport pondéral gonflant/diluant compris entre 0,2 et 0,6 et d'une quantité pondérale de principe actif comprise entre 1 et 10% par rapport au poids total du comprimé ;

3) ajout d'un lubrifiant et mélange pendant environ 10 minutes ;

4) compression sur une machine rotative de production.

A titre d'exemple, la composition de différents comprimés réalisés par le procédé selon la présente invention est indiquée ci-après dans le tableau I dans lequel toutes les valeurs numériques représentent des mg.

Les rapports pondéraux entre les différents composants de ces comprimés sont indiqués dans le tableau II.

T A B L E A U   I

| Comprimé N° | 1 | 2 | 3 | 4 |
|---|---|---|---|---|
| sulfate de salbutamol | 9,6 | 9,6 | 6,0 | 14,40 |
| Lactose | 105,0 | 150,0 | 107,0 | 135,0 |
| Phosphate dicalcique | | | | 30,0 |
| Amidon maïs prégélatinisé | 64,0 | 70,9 | 36,0 | 105,0 |
| Cellulose microfine | 15,0 | | | 10,0 |
| MHPC 0,1 Pa.s. | | | 8,0 | 40,0 |
| MHPC  4 Pa.s. | 26,0 | | | |
| MHPC  15 Pa.s. | 58,0 | 86,0 | 50,0 | 88,0 |

EP 0 299 877 B1

S U I T E   T A B L E A U   I

| Comprimé N° | 1 | 2 | 3 | 4 |
|---|---|---|---|---|
| Stéarate de Mg | 2,1 | 2,7 | 1,6 | 3,3 |
| Silice colloïdale | 2,1 | 2,7 | 1,6 | 3,3 |
| Laque EEC N° 132 | 0,1 | 0,1 | 0,06 | 0,15 |
| Poids unitaire | 281,90 | 322,0 | 210,26 | 429,15 |

EP 0 299 877 B1

<u>T A B L E A U   II</u>

| Comprimé N° | 1 | 2 | 3 | 4 |
|---|---|---|---|---|
| G/D | 0,45 | 0,38 | 0,40 | 0,45 |
| DE/G | 0,94 | 0,82 | 0,62 | 0,89 |
| DE/D | 0,42 | 0,31 | 0,25 | 0,41 |
| P.A./poids total du comprimé $\times 10^{-2}$ | 3,40 | 2,98 | 2,85 | 3,35 |
| P.A./G | 0,114 | 0,110 | 0,103 | 0,112 |
| P.A./D | 0,051 | 0,042 | 0,041 | 0,051 |

A titre d'exemple, nous donnons en outre ci-après, les résultats d'essais effectués en vue de connaître la cinétique de libération du comprimé N° 2 ; ces résultats sont donnés sous la forme des figures suivantes :

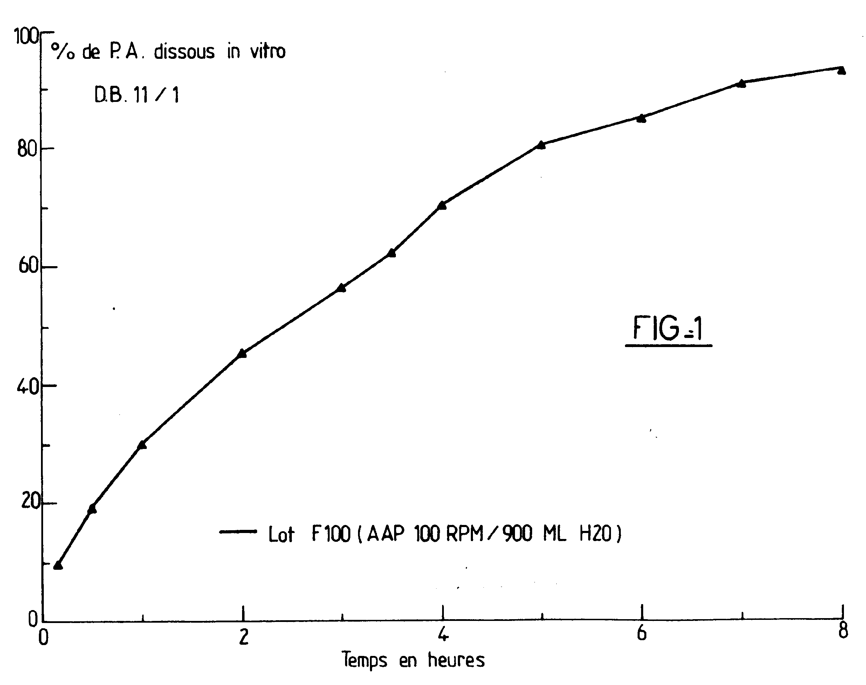

– la figure 1 correspond à la cinétique de libération in vitro et traduit l'évolution du pourcentage de principe actif dissous en fonction du temps.

L'établissement de normes de libération in vitro, a été effectué à l'aide de l'appareil à palettes tournantes (100 rpm/900 ml d'eau). Les résultats obtenus sont les suivants :

```
% dissous à 0,17 heure   :      3 %  à   18 %
% dissous à 1    heure   :     20 %  à   40 %
% dissous à 4    heures  :     60 %  à   80 %
% dissous à 8    heures  :         >  80 %
```

– La figure 2 représente une interprétation partielle de la figure 1 : on a effectué une linéarisation des pourcentages de principe actif dissous in vitro entre 0,5 h et 6 h, afin d'évoquer un flux moyen constant de libération de l'ordre de 14,21 % par heure.

Sur cette figure X représente le temps (en heures) et Y représente le pourcentage de principe actif dissous.

(Régression choisie pour la linéarisation : Y = AX + B

avec A = 12,16

et   B = 18,09

Coefficient de corrélation * r = 0,9884.).

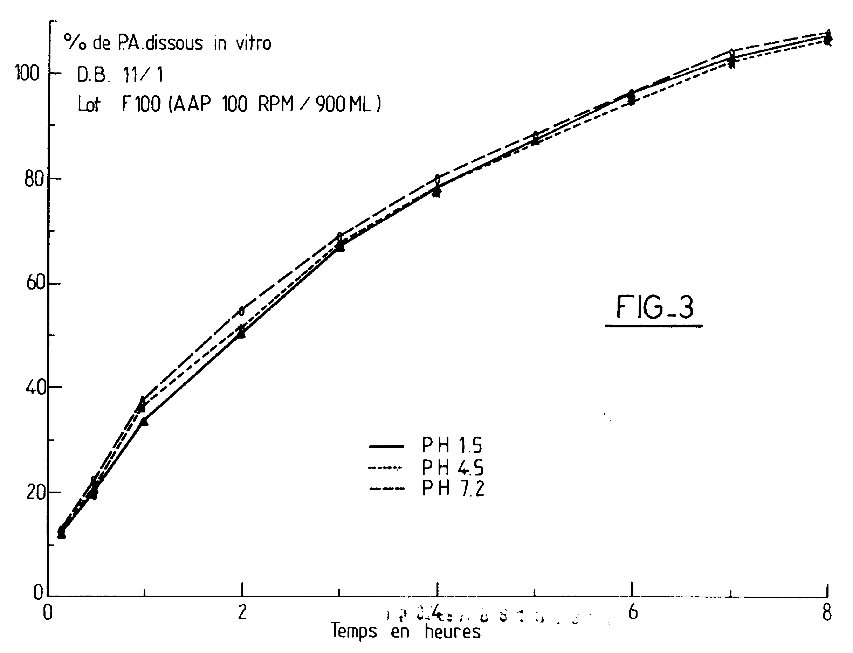

– La figure 3 représente la dissolution in vitro de principe actif en fonction de différents pH.

Sur cette figure :  _____  représente la 1ère courbe (milieu pH 1,5).

Surface sous la courbe = 574,11

(méthode des trapèzes).

•• •• •• •• ••   représente la 2ème courbe (milieu pH 4,5)

Surface sous la courbe = 573,37

(méthode des trapèzes)

- - - - - représente la 3ème courbe (milieu pH 7,2)

Surface sous la courbe = 588,49

(méthode des trapèzes).

On constate qu'il n'y a pas de différence entre les trois cinétiques de libération respectives à pH 1,5, 4,5 et 7,2.

La libération du principe actif est donc indépendante du pH.

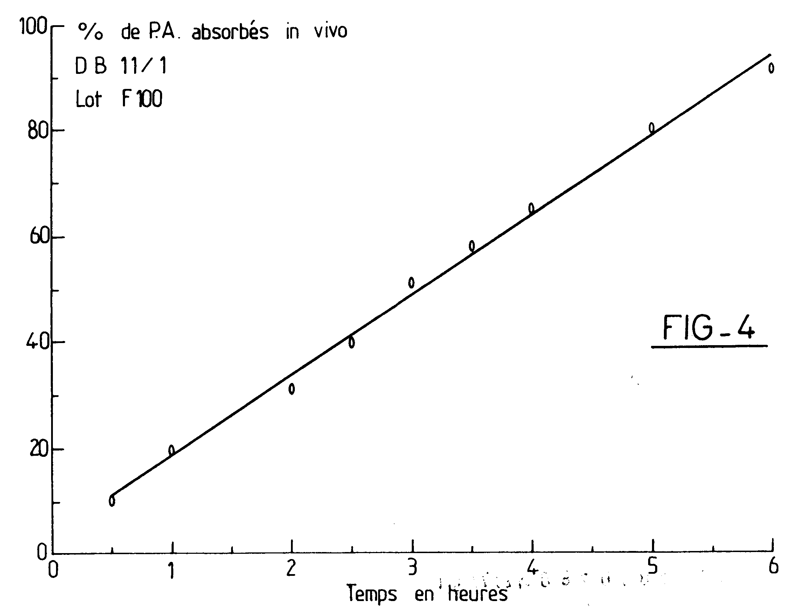

– La figure 4 concerne la cinétique d'absorption de salbutamol in vivo (linéarisation:0,5 à 6 heures) à partir d'un comprimé matrice. On obtient un flux moyen constant d'absorption in vivo.

Après administration orale des comprimés N° 2 à six sujets, les concentration plasmatiques en salbutamol ont été interprétées de façon à mettre en évidence un profil d'absorption caractérisé par une entrée d'ordre zéro jusqu'à la sixième heure suivant la prise ; à ce titre, le flux moyen constant d'absorption in vivo évoqué par la figure 4 est de l'ordre de 15,24 % par heure :

Sur cette figure X représente le temps (en heures) et Y représente le pourcentage (%) de principe actif absorbé in vivo.

(Régression-choisie : 1. Y = AX + B

avec A = 15,10

et B = 3,48

Coefficient de corrélation * r = 0,9886).

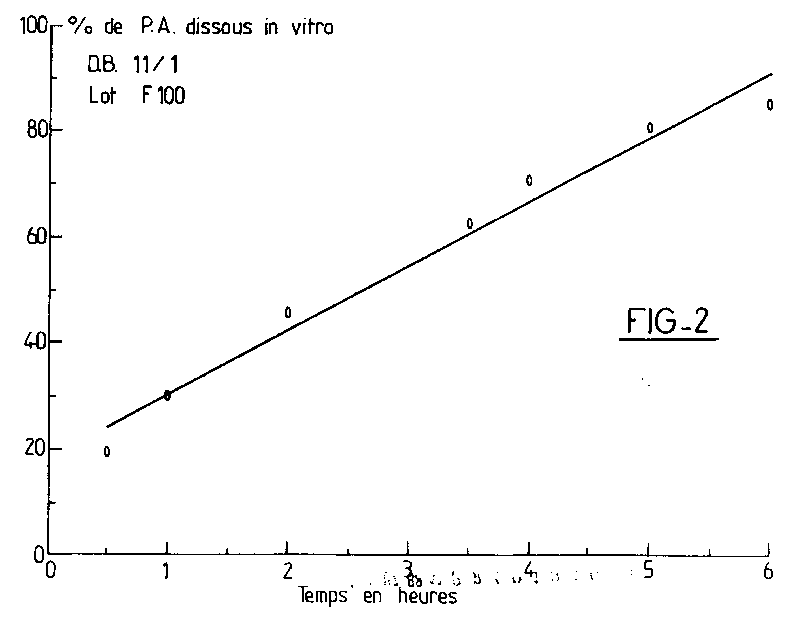

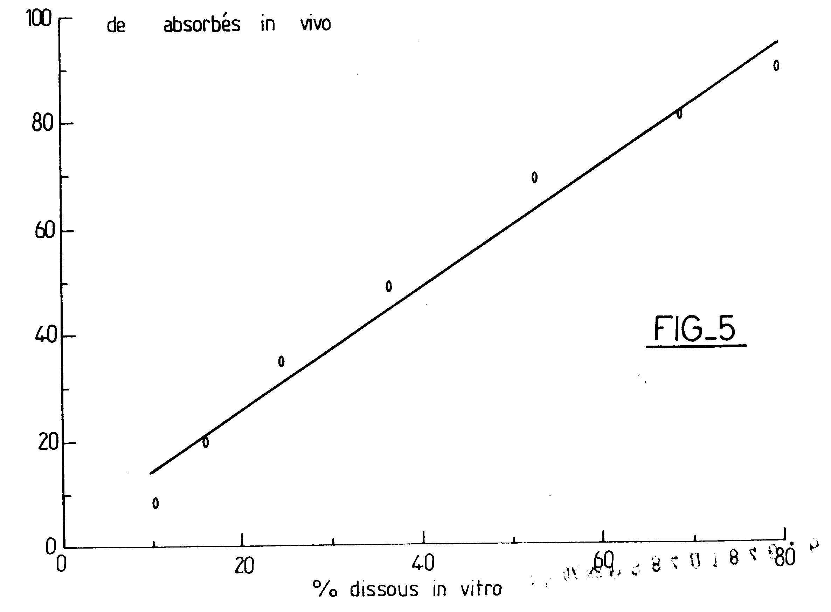

– La figure 5 représente la corrélation de cette cinétique d'absorption in vivo (figure 4) avec la cinétique de dissolution in vitro (Figure 2) : en effet, à chacun des temps de prélèvements précédents, il est possible d'associer une valeur de l'absorption in vivo (Y, en ordonnée), avec une valeur de libération par dissolution in vitro (X, en abscisse) et d'obtenir alors une corrélation significative.

Sur cette figure, X représente le pourcentage (%) dissous in vitro et Y représente le pourcentage (%) absorbé in vivo.

(Régression choisie : 1. Y = Ax + B

avec A = 1,14

et   B = 2,94

Coefficient de corrélation * r = 0,9886).

Cette corrélation significative entre les données in vitro et in vivo, les premiers résultats obtenus in vivo ainsi que la cinétique de libération in vitro confirment les propriétés avantageuses relatives à la libération du P.A. à partir des comprimés selon la présente invention.

**Revendications**

**Revendications pour les Etats contractants suivants : AT, BE, CH, DE, FR, GB, IT, LI, LU, NL, SE**

1. Comprimé du type comportant une dispersion homogène d'un principe actif à base de salbutamol ou de l'un de ses dérivés dans une matrice hydrophile, destiné à assurer
   – in vitro, une libération prolongée, régulière et indépendante du pH, du principe actif pendant un intervalle de temps d'environ 12 heures;
   – in vivo, une cinétique d'entrée du principe actif dans l'organisme d'ordre zéro pendant un intervalle de temps d'environ 6 heures, caractérisé en ce que ladite matrice hydrophile comporte :
   . à titre de composant gonflant au moins un hydrocolloïde cellulosique de haut poids moléculaire choisi parmi une ou plusieurs substances polymères hydrophiles de viscosité apparente comprise entre 0,1 et 30 Pa. s,
   . un composant diluant (D) comprenant un diluant intrinsèque (DI) choisi parmi une ou plusieurs substances comprenant le lactose, le sorbitol, le mannitol, les phosphates ou sulfates de calcium, la silice colloïdale et/ou la cellulose microcristalline, et un diluant épaississant (DE), hydrophile partiellement soluble choisi parmi une ou plusieurs substances comprenant les amidons et dérivés des amidons, en particulier l'amidon de maïs prégélatinisé,
   le rapport pondéral gonflant/diluant étant compris entre 0,2 et 0,6, de préférence égal à 0,4.

2. Comprimé selon la revendication 1, caractérisé en ce que le rapport pondéral DE/D est compris entre 0,1 et 0,6.

3. Comprimé selon l'une des revendications 1 ou 2, caractérisé en ce que le principe actif (PA) est présent à raison de 1 à 10 % en poids par rapport au poids total dudit comprimé.

4. Comprimé selon la revendication 3, caractérisé en ce que le principe actif est présent à raison de 3 % en poids par rapport au poids total dudit comprimé.

5. Comprimé selon l'une quelconque des revendications 1 à 4, caractérisé en ce que la ou les substances polymères hydrophiles sont choisies dans la famille des hydrocolloïdes protéiques ou cellulosiques.

6. Comprimé selon la revendication 5, caractérisé en ce que la ou les substances polymères sont choisies parmi les dérivés alginiques, les gommes naturelles, la méthylcellulose, la carboxyméthylcellulose, l'hydroxypropylcellulose et/ou la méthylhydroxypropylcellulose.

7. Comprimé selon la revendication 6, caractérisé en ce que la substance polymère est de la méthylhydroxypropylcellulose dont la viscosité est d'environ 15 Pa.s.

8. Comprimé selon l'une quelconque des revendications 1 à 7, caractérisé en ce qu'il comporte en outre un lubrifiant.

9. Comprimé selon la revendication 8, caractérisé en ce que le lubrifiant est l'acide stéarique ou l'un de ses dérivés tels que le stéarate de magnésium.

10. Comprimé selon l'une quelconque des revendications 1 à 9, caractérisé en ce qu'il comporte une substance colorante pharmaceutiquement acceptable telle que l'indigotine.

11. Comprimé selon l'une quelconque des revendications 1 à 10, caractérisé en ce qu'il se présente sous une forme galénique oblongue éventuellement sécable.

12. Comprimé selon l'une quelconque des revendications 1 à 11, possédant la composition suivante :

| | | |
|---|---:|---|
| – Sulfate de salbutamol | 9,6 | mg |
| – Méthylhydroxypropylcellulose, 15 Pa.s | 86 | mg |
| – Lactose | 150 | mg |
| – Amidon de maïs prégélatinisé | 70,9 | mg |
| – Silice colloïdale | 2,7 | mg |
| – Stéarate de magnésium | 2,7 | mg |
| – Indigotine EEC N° 132 | 0,1 | mg |
| pour un comprimé terminé de | 322,0 | mg |

13. Procédé de fabrication d'un comprimé selon l'une quelconque des revendications 1 à 12, caractérisé en ce qu'il comporte les opérations successives suivantes :
1) tamisage des différents composants, gonflant et diluant, et du principe actif ;
2) mélange de ces composants, gonflant et diluant, et du principe actif à raison d'un rapport pondéral gonflant/diluant compris entre 0,2 et 0,6 et d'une quantité pondérale de principe actif comprise entre 1 et 10 % par rapport au poids total du comprimé ;
3) ajout d'un lubrifiant et mélange pendant environ 10 minutes ;
4) compression sur une machine rotative de production.

**Revendications pour les Etats contractants suivants : ES, GR**

1. Procédé de préparation d'un comprimé du type comportant une dispersion homogène d'un principe actif à base de salbutamol ou de l'un de ses dérivés dans une matrice hydrophile, destiné à assurer
– in vitro, une libération prolongée, régulière et indépendante du pH, du principe actif pendant un intervalle de temps d'environ 12 heures;
– in vivo, une cinétique d'entrée du principe actif dans l'organisme d'ordre zéro pendant un intervalle de temps d'environ 6 heures, ladite matrice hydrophile comportant :
. à titre de composant gonflant au moins un hydrocolloïde cellulosique de haut poids moléculaire choisi parmi une ou plusieurs substances polymères hydrophiles de viscosité apparente comprise entre 0,1 et 30 Pa. s,
. un composant diluant (D) comprenant un diluant intrinsèque (DI) choisi parmi une ou plusieurs substances comprenant le lactose, le sorbitol, le mannitol, les phosphates ou sulfates de calcium, la silice colloïdale et/ou la cellulose microcristalline, et un diluant épaississant (DE), hydrophile partiellement soluble choisi parmi une ou plusieurs substances comprenant les amidons et dérivés des amidons, en particulier l'amidon de maïs prégélatinisé, caractérisé en ce qu'il comporte les opérations successives suivantes :
1) tamisage des différents composants, gonflant et diluant, et du principe actif ;
2) mélange de ces composants, gonflant et diluant, et du principe actif à raison d'un rapport pondéral gonflant/diluant compris entre 0,2 et 0,6, et de préférence égal à 0,4 ;
3) ajout d'un lubrifiant et mélange pendant environ 10 minutes ;
4) compression sur une machine rotative de production.
2. Procédé de préparation d'un comprimé selon la revendication 1, caractérisé en ce que le rapport pondéral DE/D est compris entre 0,1 et 0,6.
3. Procédé de préparation d'un comprimé selon l'une des revendications 1 ou 2, caractérisé en ce que le principe actif (PA) est présent à raison de 1 à 10 % en poids par rapport au poids total dudit comprimé.
4. Procédé de préparation d'un comprimé selon la revendication 3, caractérisé en ce que le principe actif est présent à raison de 3 % en poids par rapport au poids total dudit comprimé.
5. Procédé de préparation d'un comprimé selon l'une quelconque des revendications 1 à 4, caractérisé en ce que la ou les substances polymères hydrophiles sont choisies dans la famille des hydrocolloïdes protéiques ou cellulosiques.
6. Procédé de préparation d'un comprimé selon la revendication 5, caractérisé en ce que la ou les substances polymères sont choisies parmi les dérivés alginiques, les gommes naturelles, la méthylcellulose, la carboxyméthylcellulose, l'hydroxypropylcellulose et/ou la méthylhydroxypropylcellulose.
7. Procédé de préparation d'un comprimé selon la revendication 6, caractérisé en ce que la substance polymère est de la méthylhydroxypropylcellulose dont la viscosité est d'environ 15 Pa.s.
8. Procédé de préparation d'un comprimé selon l'une quelconque des revendications 1 à 7, caractérisé en ce qu'il comporte en outre un lubrifiant.

9. Procédé de préparation d'un comprimé selon la revendication 8, caractérisé en ce que le lubrifiant est l'acide stéarique ou l'un de ses dérivés tels que le stéarate de magnésium.

10. Procédé de préparation d'un comprimé selon l'une quelconque des revendications 1 à 9, caractérisé en ce qu'il comporte une substance colorante pharmaceutiquement acceptable telle que l'indigotine.

11. Procédé de préparation d'un comprimé selon l'une quelconque des revendications 1 à 10, caractérisé en ce qu'il se présente sous une forme galénique oblongue éventuellement sécable.

12. Procédé de préparation d'un comprimé selon l'une quelconque des revendications 1 à 11, possédant la composition suivante :

| | | | |
|---|---|---|---|
| - | Sulfate de salbutamol | 9,6 | mg |
| - | Méthylhydroxypropylcellulose, 15 Pa.s | 86 | mg |
| - | Lactose | 150 | mg |
| - | Amidon de maïs prégélatinisé | 70,9 | mg |
| - | Silice colloïdale | 2,7 | mg |
| - | Stéarate de magnésium | 2,7 | mg |
| - | Indigotine EEC N° 132 | 0,1 | mg |
| | pour un comprimé terminé de | 322,0 | mg |

**Patentansprüche**

**Patentanspruch für folgende Vertragsstaaten : AT, BE, CH, DE, FR, GB, IT, LI, LU, NL, SE**

1. Tablette des Typs, der umfaßt eine homogene Dispersion eines aktiven Prinzips (Wirkstoffes) auf Basis von Salbutamol oder eines seiner Derivate in einer hydrophilen Matrix, die dafür bestimmt ist, zu gewährleisten
– in vitro eine vom pH-Wert unabhängige, regelmäßige und anhaltende (verlängerte) Freisetzung des aktiven Prinzips (Wirkstoffs) während eines Zeitintervalls von etwa 12 Stunden;
– in vivo eine Aufnahmekinetik des aktiven Prinzips (Wirkstoffes) in den Organismus in der Größenordnung 0 während eines Zeitintervalls von etwa 6 Stunden, dadurch gekennzeichnet, daß die genannte hydrophile Matrix umfaßt:
. als Quellungsmittel-Bestandteil mindestens ein Cellulose-Hydrokolloid mit hohem Molekulargewicht, ausgewählt aus einer oder mehr hydrophilen polymeren Substanzen mit einer scheinbaren Viskosität zwischen 0,1 und 30 Pa.s,
. einen Verdünnungsmittel-Bestandteil (D), umfassend ein Intrinsic-Verdünnungsmittel (DI), ausgewählt aus einer oder mehr Substanzen, die umfassen Lactose, Sorbit, Mannit, die Phosphate oder Sulfate von Calcium, kolloidales Siliciumdioxid und(oder mikrokristalline Cellulose, und ein hydrophiles, teilweise lösliches Eindickungs-Verdünnungsmittel (DE), ausgewählt aus einer oder mehr Substanzen, enthaltend Stärken und Stärkederivate, insbesondere vorgelatinierte Maisstärke,
wobei das Gewichtsverhältnis Quellungsmittel/Verdünnungsmittel zwischen 0,2 und 0,6, vorzugsweise bei 0,4, liegt.

2. Tablette nach Anspruch 1, dadurch gekennzeichnet, daß das Gewichtsverhältnis DE/D zwischen 0,1 und 0,6 liegt.

3. Tablette nach einem der Ansprüche 1 oder 2, dadurch gekennzeichnet, daß das aktive Prinzip (der Wirkstoff) (PA) in einer Menge von 1 bis 10 Gew.-%, bezogen auf das Gesamtgewicht der Tablette, vorliegt.

4. Tablette nach Anspruch 3, dadurch gekennzeichnet, daß das aktive Prinzip (der Wirkstoff) in einer Menge von 3 Gew.-%, bezogen auf das Gesamtgewicht der Tablette, vorliegt.

5. Tablette nach einem der Ansprüche 1 bis 4, dadurch gekennzeichnet, daß die hydrophile(n) polymere(n) Substanz(en) ausgewählt wird (werden) aus der Familie der Protein- oder Cellulose-Hydrokolloide.

6. Tablette nach Anspruch 5, dadurch gekennzeichnet, daß die polymere(n) Substanz(en) ausgewählt wird (werden) aus den Alginderivaten, den Naturharzen (Naturgummis), Methylcellulose, Carboxymethylcellulose, Hydroxypropylcellulose und/oder Methylhydroxypropylcellulose.

7. Tablette nach Anspruch 6, dadurch gekennzeichnet, daß es sich bei der polymeren Substanz um die Methylhydroxypropylcellulose handelt, deren Viskosität etwa 15 Pa.s beträgt.

8. Tablette nach einem der Ansprüche 1 bis 7, dadurch gekennzeichnet, daß sie außerdem ein Gleitmittel enthält.

9. Tablette nach Anspruch 8, dadurch gekennzeichnet, daß es sich bei dem Gleitmittel um die Stearinsäure oder eines ihrer Derivate, wie z.B. Magnesiumstearat, handelt.

10. Tablette nach einem der Ansprüche 1 bis 9, dadurch gekennzeichnet, daß sie eine pharmazeutisch akzeptable färbende Substanz wie Indigotin enthält.

11. Tablette nach einem der Ansprüche 1 bis 10, dadurch gekennzeichnet, daß sie in einer gegebenenfalls teilbaren, länglichen galenischen Form vorliegt.

12. Tablette nach einem der Ansprüche 1 bis 11, welche die folgende Zusammensetzung aufweist:

| | | |
|---|---:|---|
| – Salbutamolsulfat | 9,6 | mg |
| – Methylhydroxypropylcellulose, 15 Pa.s | 86 | mg |
| – Lactose | 150 | mg |
| – vorgelatinierte Maisstärke | 70,9 | mg |
| – kolloidales Siliciumdioxid | 2,7 | mg |
| – Magnesiumstearat | 2,7 | mg |
| – Indigotin EEC Nr. 132 | 0,1 | mg |
| für eine fertige Tablette von | 322,0 | mg |

13. Verfahren zur Herstellung einer Tablette nach einem der Ansprüche 1 bis 12, dadurch gekennzeichnet, daß es die nachstehend angegebenen, aufeinanderfolgenden Arbeitsgänge umfaßt:

(1) das Sieben der verschiedenen Bestandteile, des Quellungsmittels und des Verdünnungsmittels und des aktiven Prinzips (des Wirkstoffes);

(2) das Mischen dieser Bestandteile, des Quellungsmittels und des Verdünnungsmittels, und des aktiven Prinzips (des Wirkstoffes) in einem Gewichtsverhältnis Quellungsmittel/Verdünnungsmittel zwischen 0,2 und 0,6 und des aktiven Prinzips (Wirkstoffes) in einer Gewichtsmenge zwischen 1 und 10 %, bezogen auf das Gesamtgewicht der Tablette;

(3) das Zugeben eines Gleitmittels und das etwa 10-minütige Mischen; und

(4) das Pressen auf einer sich drehenden Produktions-Vorrichtung.

**Patentanspruch für folgende Vertragsstaaten : ES, GR**

1. Verfahren zur Herstellung einer Tablette des Typs, die umfaßt eine homogene Dispersion eines aktiven Prinzips (Wirkstoffes) auf Basis von Salbutamol oder eines seiner Derivate in einer hydrophilen Matrix, die dafür bestimmt ist, zu gewährleisten

– in vitro eine vom pH-Wert unabhängige, regelmäßige, anhaltende (verlängerte) Freisetzung des aktiven Prinzips (Wirkstoffes) während eines Zeitintervalles von etwa 12 Stunden;

– in vivo eine Aufnahmekinetik des aktiven Prinzips (Wirkstoffes) in den Organismus in der Größenordnung 0 während eines Zeitintervalles von etwa 6 Stunden, wobei die hydrophile Matrix enthält:

– als Quellungsmittel-Komponente mindestens ein Cellulose-Hydrokolloid mit hohem Molekulargewicht, ausgewählt aus einer oder mehr hydrophilen polymeren Substanzen mit einer scheinbaren Viskosität zwischen 0,1 und 30 Pa.s,

– einen Verdünnungsmittel-Bestandteil (D), enthaltend ein Intrinsic-Verdünnungsmittel (DI), ausgewählt aus einer oder mehr Substanzen, die umfassen Lactose, Sorbit, Mannit, die Phosphate oder Sulfate von Calcium, kolloidales Siliciumdioxid und/oder mikrokristalline Cellulose, und ein hydrophiles, teilweise lösliches Eindickungs-Verdünnungsmittel (DE), ausgewählt aus einer oder mehr Substanzen, die umfassen Stärken und Stärkederivate, insbesondere vorgelatinierte Maisstärke,

dadurch gekennzeichnet, daß es die nachstehend angegebenen aufeinanderfolgenden Arbeitsgänge umfaßt:

(1) das Sieben der verschiedenen Bestandteile, des Quellungsmittels und des Verdünnungsmittels und des aktiven Prinzips (Wirkstoffes);

(2) das Mischen dieser Bestandteile, des Quellungsmittels und des Verdünnungsmittels und des aktiven Prinzips (Wirkstoffes) in einem Gewichtsverhältnis Quellungsmittel/Verdünnungsmittel zwischen 0,2 und 0,6 und vorzugsweise von 0,4;

(3) die Zugabe eines Gleitmittels und das etwa 10-minütige Mischen; und

(4) das Pressen auf einer sich drehenden Produktions-Vorrichtung.

2. Verfahren zur Herstellung einer Tablette nach Anspruch 1, dadurch gekennzeichnet, daß das Gewichtsverhältnis DE/D zwischen 0,1 und 0,6 liegt.

3. Verfahren zur Herstellung einer Tablette nach einem der Ansprüche 1 oder 2, dadurch gekennzeichnet, daß das aktive Prinzip (der Wirkstoff) (PA) in einer Menge von 1 bis 10 Gew.%, bezogen auf das Gesamtgewicht der Tablette, vorliegt.

4. Verfahren zur Herstellung einer Tablette nach Anspruch 3, dadurch gekennzeichnet, daß das aktive Prinzip (der Wirkstoff) in einer Menge von 3 Gew.-%, bezogen auf das Gesamtgewicht der Tablette, vorliegt.

5. Verfahren zur Herstellung einer Tablette nach einem der Ansprüche 1 bis 4, dadurch gekennzeichnet, daß die hydrophile(n) polymere(n) Substanz(en) ausgewählt wird (werden) aus der Familie der Protein- oder Cellulose-Hydrokolloide.

6. Verfahren zur Herstellung einer Tablette nach Anspruch 5, dadurch gekennzeichnet, daß die polymere(n) Substanz(en) ausgewählt wird (werden) aus den Alginderivaten, den Naturharzen (Naturgummis), Methylcellulose, Carboxymethylcellulose, Hydroxypropylcellulose und/oder Methylhydroxypropylcellulose.

7. Verfahren zur Herstellung einer Tablette nach Anspruch 6, dadurch gekennzeichnet, daß es sich bei der polymeren'Substanz um Methylhydroxypropylcellulose handelt, deren Viskosität etwa 15 Pa.s beträgt.

8. Verfahren zur Herstellung einer Tablette nach einem der Ansprüche 1 bis 7, dadurch gekennzeichnet, daß sie außerdem ein Gleitmittel enthält.

9. Verfahren zur Herstellung einer Tablette nach Anspruch 8, dadurch gekennzeichnet, daß es sich bei dem Gleitmittel um die Stearinsäure oder eines ihrer Derivate, wie z.B. Magnesiumstearat, handelt.

10. Verfahren zur Herstellung einer Tablette nach einem der Ansprüche 1 bis 9, dadurch gekennzeichnet, daß die Tablette eine pharmazeutisch akzeptable färbende Substanz wie Indigotin enthält.

11. Verfahren zur Herstellung einer Tablette nach einem der Ansprüche 1 bis 10, dadurch gekennzeichnet, daß die Tablette in einer gegebenenfalls teilbaren länglichen galenischen Form vorliegt.

12. Verfahren zur Herstellung einer Tablette nach einem der Ansprüche 1 bis 11, welche die folgende Zusammensetzung aufweist:

```
– Salbutamolsulfat                              9,6    mg
– Methylhydroxypropylcellulose, 15 Pa.s         86     mg
– Lactose                                       150    mg
– vorgelatinierte Maisstärke                    70,9   mg
– kolloidales Siliciumdioxid                    2,7    mg
– Magnesiumstearat                              2,7    mg
– Indigotin EEC Nr. 132                         0,1    mg
für eine fertige Tablette von                   322,0  mg
```

## Claims

## Claims for the following Contracting States : AT, BE, CH, DE, FR, GB, IT, LI, LU, NL, SE

1. Tablet of the type containing a homogeneous dispersion of an active principle based on salbutamol or one of its derivatives in a hydrophilic matrix, intended for ensuring :

– in vitro, long-term release of the active principle, which is constant and independent of pH, over a time interval of about 12 hours ;

– in vivo, uptake kinetics of the active principle by the organism of zero order over a time interval of about 6 hours, characterized in that said hydrophilic matrix comprises :

. as swelling agent, at least one high molecular weight cellulose hydrocolloid, chosen from among one or more hydrophilic polymeric substances of apparent viscosity lying between 0.1 and 30 Pa. s,

. one diluent (D) comprising one intrinsic diluent (ID) chosen from among one or more substances which include lactose, sorbitol, mannitol, the phosphates or sulphates of calcium, colloidal silica and/or microcrystalline cellulose, and one thickening diluent (TD), which is hydrophilic and partially soluble, chosen from among one or more compounds comprising starches and starch derivatives, particularly pregelatinized maize starch,

the weight of ratio swelling agent/diluent lying between 0.2 and 0.6, and being preferably equal to 0.4.

2. Tablet according to Claim 1, characterized in that the weight ratio of TD/D lies between 0.1 and 0.6.

3. Tablet according to any one of the Claims 1 or 2, characterized in that the active principle (A.P.) is present in a concentration of 1 to 10 % by weight with respect to the total weight of the tablet.

4. Tablet according to Claim 3, characterized in that the active principle is present in a concentration of 3 % by weight with the respect to the total weight of the tablet.

5. Tablet according to any one of the Claims 1 to 4, characterized in that the hydrophilic polymeric substance(s) are chosen from among the family of the cellulose and protein hydrocolloids.

6. Tablet according to Claim 5, characterized in that the polymeric substance(s) is/are chosen from among alginate derivatives, naturally occurring gums, methylcellulose, carboxymethylcellulose, hydroxypropylcellulose and/or methylhydroxypropylcellulose.

7. Tablet according to Claim 6 , characterized in that the polymeric substance is methylhydroxypropylcellulose, the viscosity of which is about 15 Pa.s.

8. Tablet according to any one of the Claims 1 to 7, characterized in that it contains in addition a lubricant.

9. Tablet according to Claim 8 , characterized in that the lubricant is stearic acid or one of its derivatives such as magnesium stearate.

10. Tablet according to any one of the Claims 1 to 9 , characterized in that it contains a pharmaceutically acceptable colouring material such as indigotin.

11. Tablet according to any one of the Claims 1 to 10, characterized in that it is available in an ablong, possibly divisible galenic form.

12. Tablet according to any one of the Claims 1 to 11, possessing the following composition :

```
–  salbutamol sulfate.....................................        9.6 mg

–  methylhydroxypropylcellulose, 15 Pa.s................         86 mg

–  lactose..............................................        150 mg

–  pregelatinized maize starch.........................        70.9 mg

–  colloidal silica.....................................        2.7 mg

–  magnesium stearate...................................        2.7 mg

–  Indigotin EEC No. 132...............................        0.1 mg

for a finished tablet of...............................       322.0 mg
```

13. Process for the manufacture of a tablet according to any one of the Claims 1 to 12, characterized in that it comprises the following sequences of steps :

1) sieving of the different components, the swelling agent, diluent and the active principle;

2) mixing of these components, the swelling agent, diluent and the active principle in a weight ratio of swelling agent/diluent lying between 0.2 and 0.6, and a weight of the active principle varying between 1 and 10 % by weight of the total weight of the tablet;

3) addition of a lubricant and mixing for about 10 minutes;

4) compression in a rotatory production machine.

**Claims for the following Contracting States : ES, GR**

1. Process for preparing a tablet of the type containing a homogeneous dispersion of an active principle based on salbutamol or one of its derivatives in a hydrophilic matrix, intended for ensuring :

– in vitro, long-term release of the active principle, which is constant and independent of pH, over a time interval of about 12 hours ;

– in vivo, uptake kinetics of the active principle by the organism of zero order over a time interval of about 6 hours, said hydrophilic matrix comprising :

. as swelling agent, at least one high molecular weight cellulose hydrocolloid, chosen from among one or more hydrophilic polymeric substances of apparent viscosity lying between 0.1 and 30 Pa. s,

. one diluent (D) comprising one intrinsic diluent (ID) chosen from among one or more substances which include lactose, sorbitol, mannitol, the phosphates or sulphates of calcium, colloidal silica and/or microcrystalline cellulose, and one thickening diluent (TD), which is hydrophilic and partially soluble, chosen from among one or more compounds comprising starches and starch derivatives, particularly pregelatinized maize starch, characterized in that it comprises the following sequence of steps :

1) sieving of the different components, the swelling agent, diluent and the active principle;

2) mixing of these components, the swelling agent, diluent and the active principle in a weight ratio of swelling agent/diluent lying between 0.2 and 0.6, and preferably equal to 0.4;

3) addition of a lubricam and mixing for about 10 minutes;

4) compression in a rotatory production machine.

2. Process for preparing a tablet according to Claim 1, characterized in that the weight ratio of TD/D lies between 0.1 and 0.6.

3. Process for preparing a tablet according to any one of the Claims 1 or 2, characterized in that the active principle (A.P.) is present in a concentration of 1 to 10 % by weight with respect to the total weight of the tablet.

4. Process for preparing a tablet according to Claim 3, characterized in that the active principle is present in a concentration of 3 % by weight with the respect to the total weight of the tablet.

5. Process for preparing a tablet according to any one of the Claims 1 to 4, characterized in that the hydrophilic polymeric substance(s) are chosen from among the family of the cellulose and protein hydrocolloids.

6. Process for preparing a tablet according to Claim 5, characterized in that the polymeric substance(s) is/are from among alginate derivatives, naturally occuring gums, methylcellulose, carboxymethylcellulose, hydroxypropylcellulose and/or methylhydroxypropylcellulose.

7. Process for preparing a tablet according to Claim 6, characterized in that the polymeric substance is methylhydroxypropylcellulose, the viscosity of which is about 15 Pa.s.

8. Process for preparing a tablet according to any one of the Claims 1 to 7, characterized in that it contains in addition a lubricant.

9. Process for preparing a tablet according to Claim 8, characterized in that the lubricant is stearic acid or one of its derivatives such as magnesium stearate.

10. Process for preparing a tablet according to any one of the Claims 1 to 9, characterized in that it contains a pharmaceutically acceptable colouring material such as indigotin.

11. Process for preparing a tablet according to any one of the Claims 1 to 10, characterized in that it is available in an ablong, possibly divisible galenic form.

12. Process for preparing a tablet according to any one of the Claims 1 to 11, possessing the following composition :

- salbutamol sulfate.................................... 9.6 mg
- methylhydroxypropylcellulose, 15 Pa.s................ 86 mg
- lactose............................................. 150 mg
- pregelatinized maize starch......................... 70.9 mg
- colloidal silica.................................... 2.7 mg
- magnesium stearate.................................. 2.7 mg
- Indigotin EEC No. 132............................... 0.1 mg

for a finished tablet of............................. 322.0 mg

% de P.A. dissous in vitro

D.B. 11 / 1

FIG.1

— Lot F100 (AAP 100 RPM / 900 ML H20)

Temps en heures

EP 0 299 877 B1

% de P.A. dissous in vitro

D.B. 11/1
Lot F 100

FIG-2

Temps en heures

EP 0 299 877 B1

FIG_3

% de P.A. dissous in vitro

D.B. 11/1

Lot F 100 (AAP 100 RPM / 900 ML)

PH 1.5
PH 4.5
PH 7.2

Temps en heures

EP 0 299 877 B1

% de P.A. absorbés in vivo

D B 11/1

Lot F 100

Temps en heures

<u>FIG-4</u>

FIG_5

EP 0 299 877 B1